# EUROPÄISCHE PATENTSCHRIFT

(11) **EP 0 761 633 B1**
(45) Veröffentlichungstag und Bekanntmachung des Hinweises auf die Patenterteilung: **15.12.1999**
(21) Anmeldenummer: 96710013.2
(22) Anmeldetag: 20.08.1996
(51) Int. Cl.: C07C 43/225, C07C 41/22

(54) **Verfahren zur Herstellung von (4-Bromphenyl-)alkylethern**
Process for the preparation of (4-bromophenyl) alkyl ethers
Procédé pour la préparation d'éthers (4-bromophényl) alkyl

(30) Priorität: 26.08.1995 DE 19531408
(43) Veröffentlichungstag der Anmeldung: 12.03.1997
(73) Patentinhaber: Clariant GmbH, 65929 Frankfurt am Main (DE)
(72) Erfinder: Wessel, Thomas, Dr., 60386 Frankfurt (DE)

(56) Entgegenhaltungen:
- WO-A-92/13820
- DE-C- 4 443 592
- SYNTHESIS, Nr. 8, August 1995, STUTTGART DE, Seiten 926-928, XP002030321 B. KRASSOWSKA-SWIEBOCKA: "Aromatic iodination of activated arenes and heterocycles with lead tetraacetate as the oxidant"

## Beschreibung

Die vorliegende Erfindung betrifft ein Verfahren zur Herstellung von (4-Bromphenyl-)alkylethern durch Bromierung der entsprechenden Alkylphenylether mit Brom in Gegenwart eines Oxidationsmittels.

(4-Bromphenyl-)alkylether finden als wertvolle Synthesebausteine eine weit verbreitete Anwendung, z. B. als Zwischenprodukte für Insektizide, zur Herstellung von Arzneistoffen sowie als Zusatzstoffe im Kunststoff- und Kautschuk-Bereich.

Bekanntlich werden diese Verbindungen durch Bromierung der entsprechenden Alkylphenylether hergestellt, wobei Alternativ-Synthesen, wie z. B. Brom-Sandmeyer-Reaktionen der entsprechend substituierten Aniline von untergeordneter Bedeutung sind (siehe z. B. Chem. Ber., 32 (1899) 160).

Bevorzugte Lösungsmittel bei der Bromierung von Alkylphenylethern sind oftmals inerte chlorierte Kohlenwasserstoffe, wie u. a. Dichlorethan, Methylenchlorid und Tetrachlorkohlenstoff oder Kohlenstoffdisulfid, welche gesundheitsschädlich, giftig oder gar cancerogen sind und deren Verwendung bei einer im technischen Maßstab erfolgenden Synthese unter ökologisch und arbeitshygienischen Aspekten bedenklich ist.

So beschreibt G. A. Olah et al. (Synthesis 1986, S. 868 - 870) Bromierungen von Anisol und Phenetol mit Bromodimethylsulfonium-Bromiden in Methylenchlorid als Lösungsmittel. Verfahren zur Herstellung von Bromphenolen und seinen Derivaten in Gegenwart flüssiger organischer Ester als Lösungsmittel, wie in der DE-A 37 42 515 erwähnt, erfordern bei einer Übertragung in den technischen Maßstab aus Sicherheitsgründen ebenfalls aufwendige apparative Maßnahmen, insbesondere für den Brand- und Explosionsschutz einer Anlage.

Bromierungen von Anisol bzw. Phenetol in Essigsäure sind ebenfalls bekannt, finden bislang aber nur untergeordnete Beachtung, da u. a. zur Zurückdrängung von Nebenreaktionen, wie Etherspaltung, bei der Bromierung von Phenolethern oft der Zusatz eines bromwasserstoffbindenden Mittels, wie Natriumacetat oder Calciumcarbonat oder das Austreiben der gebildeten HBr erforderlich ist (vgl. A. Roedig in Houben-Weyl-Müller, Methoden der Organischen Chemie, Band V/4 S. 246, S. 269, Stuttgart 1960). So beschreibt A. Roedig die Bromierung von Anisol in Eisessig im Luftstrom mit dampfförmigen Brom, wobei pro Mol Anisol 1.05 Mol Brom (Br₂) eingesetzt wird. Die erzielte Ausbeute beträgt 83 % d. Th. Bei diesen Verfahren fällt zwangsweise 1 Mol Bromwasserstoff pro Mol entstandenem Bromphenylalkylether an, der im Filtrat gelöst ist oder aus der Abluft herausgewaschen werden muß. Weitere beschriebene Verfahren zur Bromierung von Anisol in Eisessig (vgl. Chem. Ber., 63 (1930) 3029 und C. R. Hebd. Seances Acad. Sci., 136 (1903) 378) bzw. von Phenetol in Eisessig (ibd. 136 (1903) 378) stellen bzgl. des Bromwasserstoffanfalls keine Verbesserung dar, da bei diesen Herstellungsverfahren deutlich weniger als die Hälfte des eingesetzten Broms ausgenutzt wird.

Bromierungsreaktionen an Aromaten, bei denen der aus dem Brom entstehende Bromwasserstoff durch Zusatz eines Oxidationsmittels wieder in Brom übergeführt wird, sind bekannt. So beschreibt die EP-B1 429 975 die Herstellung von dibromierten Diphenylethern mit Brom in Gegenwart von Oxidationsmittel im 2-Phasen-System, wobei die Reaktion in Wasser nicht-mischbaren organischen Lösungsmitteln durchgeführt wird. Bevorzugte Lösungsmittel sind hierbei 1,2-Dichlorethan, Methylenchlorid oder Methylenbromid, deren Verwendung, wie bereits angeführt, unter ökologischen und arbeitshygienischen Aspekten bedenklich ist.

Synthesis, Nr.8, August 1995, Seiten 926 bis 928 beschreibt auf Seite 928 c) linke Spalte die Bromierung von Anisol in Eisessig in gegenwart von Pb (IV) - acetat. Die Verwendung von Pb (IV) - acetat ist wegen der Giftigkeit von Blei problematisch und führt bei der Entsorgung zu Schwierigkeiten, da Blei als toxisches Schwermetall in Ruckständen aller Art in hohem Maße unerwünscht ist.

Weiterhin ist von B. M. Choudary et al. (Synlett 1994, S. 450) die regioselektive Oxybromierung u. a. von Anisol in Gegenwart von Ammoniummolybdat-Katalysatoren beschrieben. Verwendetes Lösungsmittel ist hierbei zwar ökologisch unbedenkliche Essigsäure, aber durch den Einsatz eines Schwermetall-Katalysators bedingt dieses Verfahren bei Übertragung in den technischen Maßstab erhebliche Entsorgungsprobleme, zudem die Verwendung eines nicht regenerierbaren Katalysators einen nicht unbedeutenden Preisnachteil darstellt. Durch den Einsatz von Kaliumbromid fallen zusätzlich bei diesem Verfahren große Mengen an anorganischen Salzen an, die aus Prozeßabwässern arbeits- und energieaufwendig entfernt werden müssen.

Aufgabe der vorliegenden Erfindung ist es daher, ein unter ökologisch und arbeitshygienischen Aspekten verbessertes, die Abwasserbelastung verringerndes und die Verwendung von chlorierte Kohlenwasserstoffen vermeidendes Verfahren zur Herstellung von (4-Bromphenyl-)alkylethern bereitzustellen, das in technisch einfacher Weise in hoher Ausbeute hochreine Produkte liefert.

Die vorliegende Erfindung betrifft somit ein Verfahren zur Herstellung von Verbindungen der allgemeinen Formel I 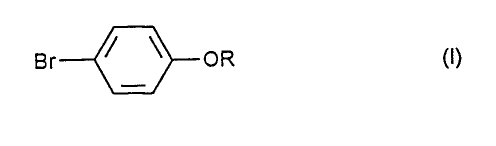 worin R für eine Alkylgruppe steht, durch Umsetzung einer Verbindung der allgemeinen Formel II 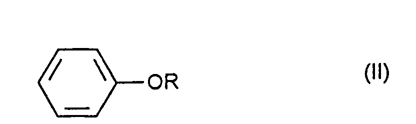 worin R wie oben angegeben definiert ist, mit Brom in einem wassermischbaren Lösungsmittel, dadurch gekennzeichnet, daß in Gegenwart von Braunstein, eines Cer (IV) - salzes, eines Alkalimetallbromats oder einer Peroxyverbindung als Oxidationsmittel, das Bromwasserstoff zu Brom zu oxidieren vermag, und einer wassermischbaren Carbonsäure gearbeitet wird.

Für R stehende Alkylgruppen können geradkettig oder verzweigt sein und haben bevorzugt ein bis sechs Kohlenstoffatome. Beispiele sind Methyl, Ethyl, n-Propyl, i-Propyl, n-Butyl, i-Butyl, sek. Butyl und tert. Butyl. Besonders bevorzugt steht R für Methyl und Ethyl.

Geeignete Oxidationsmittel, die Bromwasserstoff zu Brom zu oxidieren vermögen, sind insbesondere solche, die weder mit den Verbindungen der allgemeinen Formel I noch mit den Verbindungen der allgemeinen Formel II Nebenreaktionen eingehen. Geeignete Oxidationsmittel sind wie zuvor erwahnt Braunstein, Cer(IV)-Salze, Alkalimetallbromate und Peroxyverbindungen. Bevorzugte Oxidationsmittel sind Peroxyverbindungen, worunter Wasserstoffperoxid und seine anorganischen und organischen Derivate, in denen eines oder beide Wasserstoffatome durch kovalent gebundene Reste oder durch ionisch gebundene Kationen ersetzt sind, zu verstehen sind.

Geeignete Peroxyverbindungen sind beispielsweise Natriumperoxid und Bariumperoxid sowie deren Hydrate; Butylhydroperoxid; Peroxyameisensäure, Peroxyessigsäure, Peroxypropionsäure, Peroxylaurinsäure, Peroxybenzoesäure, m-Chlorperoxybenzoesäure sowie deren Alkali- und Erdalkalisalze; die sogenannten Alkalimetallperborate und -percarbonate wie Natriumperborat und Natriumpercarbonat; sowie Peroxmonoschwefelsäure, Peroxodischwefelsäure und deren Alkali- und Ammoniumsalze.

Ein besonders bevorzugtes Oxidationsmittel ist Wasserstoffperoxid, das vorteilhafterweise in Form handelsüblicher wäßriger Lösungen im Konzentrationsbereich von 20 bis 50 Gew% eingesetzt wird.

Die benötigte Menge an Oxidationsmittel hängt insbesondere von der Reaktionsführung ab.

Häufig wird ein Überschuß des Oxidationsmittels eingesetzt, um eine vollständige und schnelle Rückoxidation zu erreichen. Wird Wasserstoffperoxid als Oxidationsmittel eingesetzt, so kann eine 0,5fache bis äquimolare molare Menge, bezogen auf die Verbindung der allgemeinen Formel II zweckmäßig sein, wobei 0,62 bis 0,72 Mol Wasserstoffperoxid, bezogen auf die Verbindung der allgemeinen Formel II besonders bevorzugt ist.

Im erfindungsgemäßen Verfahren wird Brom in der Regel in Mengen von 0,5 bis 0,6 Mol (Br₂) pro Mol Verbindung der allgemeinen Formel II eingesetzt. Besonders bevorzugt sind, je nach Ausführung des erfindungsgemäßen Verfahrens, 0,52 bis 0,56 Mol pro Mol Verbindung der allgemeinen Formel II.

Das erfindungsgemäße Verfahren wird in einem wassermischbaren Lösungsmittel oder in Mischungen wassermischbarer Lösungsmittel durchgeführt. Beispiele sind wassermischbare Carbonsäuren oder Wasser selbst. Insbesondere wenn das Oxidationsmittel in Form einer wäßrigen Lösung eingesetzt wird, ist die Verwendung wassermischbarer Carbonsäuren als Lösungsmittel bevorzugt. Beispiele für solche Carbonsäuren sind Ameisensäure, Essigsäure und Propionsäure. Ein ganz besonders bevorzugtes Lösungsmittel ist Essigsäure.

Das erfindungsgemäße Verfahren wird insbesondere bei Temperaturen zwischen 0 °C und dem Siedepunkt des Reaktionsgemisches durchgeführt. Bevorzugte Temperaturen sind 20 bis 50 °C, besonders bevorzugte Temperaturen sind 30 bis 40 °C.

In einer Ausführungsform des erfindungsgemäßen Verfahrens wird die Verbindung der allgemeinen Formel II zusammen mit dem Lösungsmittel und dem Oxidationsmittel vorgelegt und das Brom zudosiert. Es kann aber auch die Verbindung der allgemeinen Formel II zusammen mit dem Lösungsmittel vorgelegt und dann zuerst Brom und dann Oxidationsmittel zudosiert werden. Brom und Oxidationsmittel können aber auch gemeinsam zudosiert werden.

Die Isolierung der Verbindung der allgmeinen Formel I kann nach üblichen Aufarbeitungsverfahren erfolgen, die von der Löslichkeit des Produktes im verwendeten Lösungsmittel und dessen Festpunkt abhängen. Übliche Aufarbeitungsverfahren sind hierbei Chromatographie, Filtration, Phasentrennung, Zentrifugation oder Destillation bei Normaldruck oder im Vakuum. Insbesondere die destillative Aufarbeitung ist bei niederen (4-Bromphenyl-)alkylethern die Methode der Wahl.

### Beispiele

### 1. Bromierung von Phenetol in Essigsäure

In einem 1l-Mehrhalskolben mit Rührer werden 840 g Eisessig (800 ml) und 123 g Phenetol (99 %ig nach GC, entsprechend 1 mol) bei Raumtemperatur unter Zugabe von 65 g 35 %iger wäßriger Wasserstoffperoxid-Lösung (entsprechend 0,67 mol H₂O₂) miteinander vermischt. Anschließend dosiert man über einen Zeitraum von ca. 45 min 86,5 g Brom (entsprechend 0,54 mol Br₂) so zu, daß die Innentemperatur 30 - 35 °C nicht übersteigt. Die Außenkühlung erfolgt dabei mit Hilfe eines Wasserbades. Nach erfolgter Bromzugabe wird 15 min nachgerührt und sodann die Essigsäure über eine Destillationsbrücke mit Füllkörperkolonne(20 cm Höhe, Füllung: 6 mm Raschig-Ringe) i. Vak. bei 165 - 150 mbar abdestilliert.

Die abdestillierte wäßrige Essigsäure enthält 8 g 4-Bromphenetol, entsprechend 4 % d. Th.

Nach Ausdestillieren der Essigsäure erfolgt direkt die Produktdestillation i. Vak. bei 40 - 45 mbar, wobei 4-Bromphenetol konst. bei 124 - 127 °C siedet.

Man erhält 181 g 4-Bromphenetol als farbloses Öl, entsprechend 89,7 % d. Th.

| | | |
|---|---|---|
| Reingehalt (GC): | 0,53 % | 2-Bromphenetol |
| | 98,64 % | 4-Bromphenetol |
| | 0,58 % | 2,4-Dibromphenetol |

### 2. Bromierung von Phenetol in Essigsäure

Arbeitet man wie im Beispiel 1, wobei die Produktdestillation aber zusätzlich über einen Rücklaufteiler erfolgt (Rücklaufverhältnis 10 : 1), kann der Anteil an 2,4-Dibromphenetol noch deutlich verkleinert werden:

| | | |
|---|---|---|
| Reingehalt (GC): | 0,49 % | 2-Bromphenetol |
| | 98,86 % | 4-Bromphenetol |
| | 0,19 % | 2,4-Dibromphenetol |

### 3. Bromierung von Anisol in Essigsäure

In einem 1l-Mehrhalskolben mit Rührer werden 525 g Essigsäure (500 ml) und 109 g Anisol (99 %ig nach GC, entsprechend 1 mol) bei Raumtemperatur mit 65 g 35 %iger wäßriger Wasserstoffperoxid-Lösung (entsprechend 0,67 mol H₂0₂) vermischt.

Anschließend dosiert man über einen Zeitraum von ca. 1 h 84 g Brom (0,525 mol Br₂) so zu, daß die Innentemperatur 35 °C nicht übersteigt. Nach der Bromdosierung wird 30 min nachgerührt und sodann die Essigsäure über eine Destillationsbrücke mit Füllkörperkolonne (Höhe: 20 cm, 6 mm Raschig-Ringe) i. Vak. bei 157 - 167 mbar abdestilliert.

Die abdestillierte Essigsäure enthält 6,3 g 4-Bromanisol, entsprechend 3,5 % d. Th.

Nach Ausdestillieren der Essigsäure erfolgt direkt die Produktdestillation i. Vak. bei 48 - 53 mbar, wobei 4-Bromanisol konst. bei 117 - 119 °C siedet.

Man erhält so 165 g 4-Bromanisol als farbloses Öl, entsprechend 88,2 % d. Th.

| | | |
|---|---|---|
| Reingehalt (GC): | 0 % | 2-Bromanisol |
| | 99,6 % | 4-Bromanisol |
| | 0,33 % | 2,4-Dibromanisol |

## Patentansprüche

1. Verfahren zur Herstellung von Verbindungen der allgemeinen Formel I 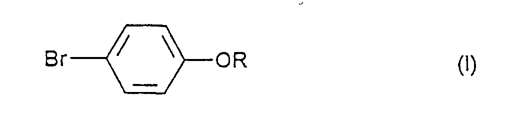 worin R für eine Alkylgruppe steht, durch Umsetzung einer Verbindung der allgemeinen Formel II 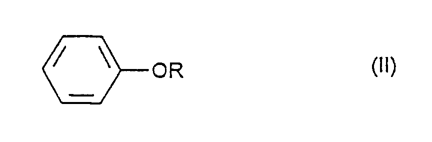 worin R wie oben angegeben definiert ist, mit Brom in einem wassermischbaren Lösungsmittel, dadurch gekennzeichnet, daß in Gegenwart von Braunstein, eines Cer(IV)-salzes, ein es Alkalimetallbromats oder einer Peroxyverbindung als Oxidationsmittelt, das Bromwasserstoff zu Brom zu oxidieren vermag, und einer wassermischbaren Carbosäure gearbeitet wird.

2. Verfahren, gemäß Anspruch 1, dadurch gekennzeichnet, daß R für (C₁-C₆)-Alkyl steht.

3. Verfahren gemäß Anspruch 1 und/oder 2, dadurch gekennzeichnet, daß R für Methyl oder Ethyl steht.

4. Verfahren gemäß einem oder mehreren der Ansprüche 1 - 3, dadurch gekennzeichnet, daß als Oxidationsmittel Wasserstoffperoxid eingesetzt wird.

5. Verfahren gemäß einem oder mehreren der Ansprüche 1 - 4, dadurch gekennzeichnet, daß als Oxidationsmittel Wasserstoffperoxid in Mengen von 0,62 bis 0,72 Mol pro Mol Verbindung der allgemeinen Formel II eingesetzt wird.

6. Verfahren gemäß einem oder mehreren der Ansprüche 1 bis 5, dadurch gekennzeichnet, daß als Lösungsmittel Essigsäure eingesetzt wird.

7. Verfahren gemäß einem oder mehreren der Ansprüche 1 bis 6, dadurch gekennzeichnet, daß Brom in Mengen von 0,5 bis 0,6 Mol (Br₂) pro Mol Verbindung der allgemeinen Formel II eingesetzt wird.

8. Verfahren gemäß einem oder mehreren der Ansprüche 1 bis 7, dadurch gekennzeichnet, daß es bei Temperaturen von 20 bis 50 °C ausgeführt wird.

## Claims

1. A process for preparing compounds of the formula I 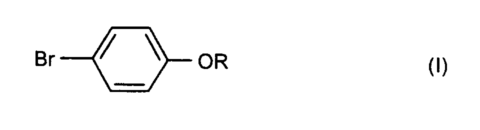 in which R is an alkyl group, by reacting a compound of the formula II 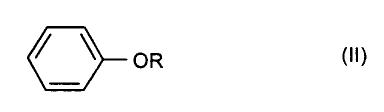 in which R is defined as specified above, with bromine in a water-miscible solvent, which comprises carrying out the process in the presence of manganese dioxide, a cerium(IV) salt, an alkali metal bromate or a peroxy compound as oxidizing agent which is able to oxidize hydrogen bromide to bromine, and a water-miscible carboxylic acid.

2. The process as claimed in claim 1, wherein R is (C₁-C₆)-alkyl.

3. The process as claimed in claim 1 and/or 2, wherein R is methyl or ethyl.

4. The process as claimed in one or more of claims 1 - 3, wherein the oxidising agent used is hydrogen peroxide.

5. The process as claimed in one or more of claims 1 - 4, wherein the oxidizing agent used is hydrogen peroxide in amounts of 0.62 to 0.72 mol per mole of compound of the formula II.

6. The process as claimed in one or more of claims 1 to 5, wherein the solvent used is acetic acid.

7. The process as claimed in one or more of claims 1 to 6, wherein bromine is used in amounts of 0.5 to 0.6 mol (Br₂) per mole of compound of the formula II.

8. The process as claimed in one or more of claims 1 to 7, wherein it is carried out at temperatures of 20 to 50°C.

## Revendications

1. Procédé pour la préparation de composés de formule générale I 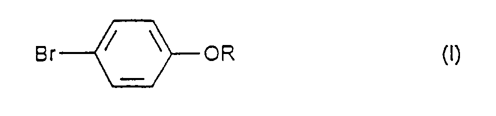 dans laquelle R représente un groupe alkyle, en mettant à réagir un composé de formule générale II 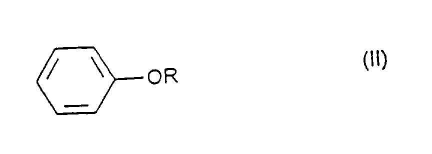 dans laquelle R est défini comme indiqué ci-dessus, avec du brome dans un solvant miscible à l'eau, caractérisé en ce qu'il est possible en présence de bioxyde de manganèse, d'un sel de Ce (IV), d'un bromate de métal alcalin ou d'un composé peroxy comme oxydant, d'oxyder le bromure d'hydrogène en brome, et en ce que le traitement est effectué dans un acide carboxylique miscible à l'eau.

2. Procédé selon la revendication 1, caractérisé en ce que R représente un groupe alkyle en C₁-C₆.

3. Procédé selon la revendication 1 et/ou 2, caractérisé en ce que R représente le groupe méthyle ou éthyle.

4. Procédé selon une ou plusieurs des revendications 1 - 3, caractérisé en ce qu'on utilise comme oxydant le peroxyde d'hydrogène.

5. Procédé selon une ou plusieurs des revendications 1 - 4, caractérisé en ce qu'on utilise comme oxydant le peroxyde d'hydrogène en quantités de 0,62 à 0,72 mol par mol de composé de formule générale II.

6. Procédé selon une ou plusieurs des revendications 1 - 5, caractérisé en ce qu'on utilise comme solvant l'acide acétique.

7. Procédé selon une ou plusieurs des revendications 1 - 6, caractérisé en ce qu'on utilise le brome en quantités de 0,5 à 0,6 mol (Br₂) par mol de composé de formule générale II.

8. Procédé selon une ou plusieurs des revendications 1 - 7, caractérisé en ce qu'il est réalisé à des températures de 20 à 50°C.
